# EUROPEAN PATENT APPLICATION

(11) **EP 3 528 261 A1**
(43) Date of publication of application: **21.08.2019**
(21) Application number: 19155430.2
(22) Date of filing: 05.02.2019
(51) Int. Cl.: G16H 50/20, G16H 30/00

(54) **PREDICTION MODEL FOR GROUPING HEPATOCELLULAR CARCINOMA, PREDICTION SYSTEM THEREOF, AND METHOD FOR DETERMINING HEPATOCELLULAR CARCINOMA GROUP**

(30) Priority: 14.02.2018 TW 107105648
(71) Applicant: China Medical University Hospital, Taichung City 404 (TW)
(72) Inventor: HUANG, Tzung-Chi, 404 Taichung City (TW); YU, Jiaxin, 408 Taichung City (TW); LIN, Yang-Hsien, 600 Chiayi City (TW); LIAO, Ken Ying-Kai, Taichung City (TW); LIN, Wei-Ching, 404 Taichung City (TW); ZHANG, Geoffrey G., Tampa, 33647 (US)
(74) Representative: Kuttenkeuler, David

(57) **Abstract**

A prediction system for grouping hepatocellular carcinoma includes an image capturing unit and a non-transitory machine readable medium. The non-transitory machine readable medium storing a program which, when executed by at least one processing unit, predicts a hepatocellular carcinoma group of the subject patient with hepatocellular carcinoma. The program includes a reference database obtaining module, a first image preprocessing module, a feature selecting module, a classifying module, a second image preprocessing module and a comparing module.

## Description

### BACKGROUND

### Technical Field

The present disclosure relates to a medical information analysis model, system and method thereof. More particularly, the present disclosure relates to a prediction model for grouping hepatocellular carcinoma, a prediction system for grouping hepatocellular carcinoma, and a method for determining hepatocellular carcinoma group.

### Description of Related Art

Hepatocellular carcinoma refers to malignant tumors that occur in or start from the liver. Symptoms of hepatocellular carcinoma may include a lump or pain in the right side below the rib cage, swelling of the abdomen, yellowish skin, easy bruising, weight loss, and weakness. The leading cause of hepatocellular carcinoma is chronic hepatitis B and chronic hepatitis C, wherein about 60% to 70% of hepatocellular carcinoma is caused by chronic hepatitis B, and about 20% of hepatocellular carcinoma is caused by chronic hepatitis C. After infection with the hepatitis B virus or hepatitis C virus, some people will become chronic hepatitis, wherein chronic hepatitis may become cirrhosis, and then cirrhosis would cause hepatocellular carcinoma.

The current diagnosis of hepatocellular carcinoma includes the following methods. First, detecting whether the subject has chronic hepatitis B or chronic hepatitis C. Second, detecting whether Alpha-Fetoprotein (AFP) index of the subject is elevated (especially above 400 ng/dL). Third, computed tomography, angiography, or magnetic resonance photography can represent typical images of hepatocellular carcinoma. If two conditions of the three methods are satisfied, hepatocellular carcinoma can be diagnosed. Furthermore, the subject who is not easy to diagnose can diagnose with liver slices. However, the aforementioned diagnosis methods only can determine whether the subject has hepatocellular carcinoma, and cannot be used to predict prognosis of the patient with hepatocellular carcinoma, or to group the patient with hepatocellular carcinoma for further providing more accurate treatment.

It can be seen that the conventional technology lacks tools with high index, better sensitivity, and can be applied to clinical hepatocellular carcinoma grouping and prognostic analysis. Therefore, it is necessary to improve the conventional technology to accurately predict the prognosis of the patients with hepatocellular carcinoma and improve their mortality.

### SUMMARY

According to one aspect of the present disclosure, a prediction model for grouping hepatocellular carcinoma includes following establishing steps. A reference database is obtained, wherein the reference database is established by a plurality of reference arterial phase computed tomographic images of a plurality of reference patients with hepatocellular carcinoma. An image preprocessing step is performed, wherein the image preprocessing step is for circling a tumor position in each of the reference arterial phase computed tomographic images to obtain reference region of interest images. A feature selecting step is performed, wherein the feature selecting step is for selecting at least one eigenvalue according to the reference database, the eigenvalue including a texture feature value after analyzing the reference region of interest images by using a Laws filter. A classifying step is performed, wherein the classifying step is for achieving a convergence of the eigenvalue by using a supervised learning method to obtain the prediction model for grouping hepatocellular carcinoma. The prediction model for grouping hepatocellular carcinoma is used to predict whether a tumor of a subject patient with hepatocellular carcinoma is metastasized, whether a metastatic site of the subject patient with hepatocellular carcinoma is in an abdominal cavity and whether the subject patient with hepatocellular carcinoma carries hepatitis viruses.

In one example, the supervised learning method can be a support vector machine.

In one example, when the prediction model for grouping the hepatocellular carcinoma is used to predict whether the tumor of the subject patient with the hepatocellular carcinoma is metastasized, the texture feature value can include a total gray-level value (TGV), a contrast, a mean, an entropy and a homogeneity of the reference region of interest image.

In one example, when the prediction model for grouping the hepatocellular carcinoma is used to predict whether the tumor of the subject patient with the hepatocellular carcinoma is metastasized, the eigenvalue selected in the feature selecting step can further include a homogeneity of the reference region of interest image calculated by a Wavelet filter and a coarseness of the reference region of interest image calculated by a Neighbor Gray Tone Difference Matrix (NGTDM) filter.

In one example, when the prediction model for grouping the hepatocellular carcinoma is used to predict whether the metastatic site of the subject patient with the hepatocellular carcinoma is in the abdominal cavity, the texture feature value can include a homogeneity and a contrast of the reference region of interest image.

In one example, when the prediction model for grouping the hepatocellular carcinoma is used to predict whether the metastatic site of the subject patient with the hepatocellular carcinoma is in the abdominal cavity, the eigenvalue selected in the feature selecting step can further include a correlation of the reference region of interest image calculated by a Gary Level Co-Occurrence Matrix (GLCM) filter.

In one example, the hepatitis viruses can be hepatitis B viruses or hepatitis C viruses.

In one example, when the prediction model for grouping the hepatocellular carcinoma is used to predict whether the subject patient with the hepatocellular carcinoma carries hepatitis viruses, the texture feature value can include a homogeneity of the reference region of interest image.

In one example, when the prediction model for grouping the hepatocellular carcinoma is used to predict whether the subject patient with the hepatocellular carcinoma carries hepatitis viruses, the eigenvalue selected in the feature selecting step can further include a minimum and a homogeneity of the reference region of interest image calculated by a Laplacian of Gaussian (LoG) filter.

According to another aspect of the present disclosure, a method for determining a hepatocellular carcinoma group includes following steps. The prediction model for grouping hepatocellular carcinoma of the aforementioned aspect is provided. A target arterial phase computed tomographic image of a subject patient with hepatocellular carcinoma is provided. A tumor position in the target arterial phase computed tomographic image is circled to obtain a target region of interest image. The prediction model for grouping hepatocellular carcinoma is used to analyze the target region of interest image to determine whether a tumor of the subject patient with hepatocellular carcinoma is metastasized, whether the metastatic position of the subject patient with hepatocellular carcinoma is in an abdominal cavity and whether the subject patient with hepatocellular carcinoma carries hepatitis viruses.

In one example, an assessment time of a tumor metastasis in the subject patient with the hepatocellular carcinoma can be within 5 years.

In one example, the hepatitis viruses can be hepatitis B viruses or hepatitis C viruses.

According to still another aspect of the present disclosure, a prediction system for grouping hepatocellular carcinoma includes an image capturing unit and a non-transitory machine readable medium. The image capturing unit is for obtaining a target arterial phase computed tomographic image of a subject patient with hepatocellular carcinoma. The non-transitory machine readable medium storing a program which, when executed by at least one processing unit, predicts a hepatocellular carcinoma group of the subject patient with hepatocellular carcinoma. The program includes a reference database obtaining module, a first image preprocessing module, a feature selecting module, a classifying module, a second image preprocessing module and a comparing module. The reference database obtaining module is for obtaining a reference database, wherein the reference database is established by a plurality of reference arterial phase computed tomographic images of a plurality of reference patients with hepatocellular carcinoma. The first image preprocessing module is for circling a tumor position in each of the reference arterial phase computed tomographic images to obtain reference region of interest images. The feature selecting module is for selecting at least one eigenvalue according to the reference database, wherein the eigenvalue includes a texture feature value after analyzing the reference region of interest images by using a Laws filter. The classifying module is for achieving a convergence of the eigenvalue by using a supervised learning method to obtain a prediction model for grouping hepatocellular carcinoma. The second image preprocessing module is for circling a tumor position in the target arterial phase computed tomographic image to obtain a target region of interest image. The comparing module is for analyzing the target region of interest image by the prediction model for grouping hepatocellular carcinoma to determine whether a tumor of the subject patient with hepatocellular carcinoma is metastasized, whether a metastatic site of the subject patient with hepatocellular carcinoma is in an abdominal cavity and whether the subject patient with hepatocellular carcinoma carries hepatitis viruses.

In one example, when the prediction system for grouping the hepatocellular carcinoma is used to determine whether the tumor of the subject patient with the hepatocellular carcinoma is metastasized, the texture feature value can include a total gray-level value (TGV), a contrast, a mean, an entropy and a homogeneity of the reference region of interest image.

In one example, when the prediction system for grouping the hepatocellular carcinoma is used to determine whether the tumor of the subject patient with the hepatocellular carcinoma is metastasized, the eigenvalue selected by the feature selecting module can further include a homogeneity of the reference region of interest image calculated by a Wavelet filter and a coarseness of the reference region of interest image calculated by a Neighbor Gray Tone Difference Matrix (NGTDM) filter.

In one example, when the prediction system for grouping the hepatocellular carcinoma is used to determine whether the metastatic site of the subject patient with the hepatocellular carcinoma is in the abdominal cavity, the texture feature value can include a homogeneity and a contrast of the reference region of interest image.

In one example, when the prediction system for grouping the hepatocellular carcinoma is used to determine whether the metastatic site of the subject patient with the hepatocellular carcinoma is in the abdominal cavity, the eigenvalue selected by the feature selecting module can further include a correlation of the reference region of interest image calculated by a Gary Level Co-Occurrence Matrix (GLCM) filter.

In one example, the hepatitis viruses can be hepatitis B viruses or hepatitis C viruses.

In one example, when the prediction system for grouping the hepatocellular carcinoma is used to determine whether the subject patient with the hepatocellular carcinoma carries hepatitis viruses, the texture feature value can include a homogeneity of the reference region of interest image.

In one example, when the prediction system for grouping the hepatocellular carcinoma is used to determine whether the subject patient with the hepatocellular carcinoma carries hepatitis viruses, the eigenvalue selected by the feature selecting module can further include a minimum and a homogeneity of the reference region of interest image calculated by a Laplacian of Gaussian (LoG) filter.

### BRIEF DESCRIPTION OF THE DRAWINGS

The present disclosure can be more fully understood by reading the following detailed description of the embodiment, with reference made to the accompanying drawings as follows:
Fig. 1 is a flowchart of establishing steps of a prediction model for grouping hepatocellular carcinoma according to one embodiment of the present disclosure.
Fig. 2 is a flowchart of a method for determining a hepatocellular carcinoma group according to another embodiment of the present disclosure.
Fig. 3 is a block diagram of a prediction system for grouping hepatocellular carcinoma according to still another embodiment of the present disclosure.
Fig. 4A shows an analysis result of a number of a feature selection for establishing a prediction model for grouping hepatocellular carcinoma used to predict whether a tumor of a subject patient with hepatocellular carcinoma is metastasized.
Fig. 4B shows a receiver operating characteristic curve (ROC) diagram of the prediction model for grouping hepatocellular carcinoma used to predict whether the tumor of the subject patient with hepatocellular carcinoma is metastasized before a feature selecting step.
Fig. 4C shows a receiver operating characteristic curve diagram of the prediction model for grouping hepatocellular carcinoma used to predict whether the tumor of the subject patient with hepatocellular carcinoma is metastasized after the feature selecting step.
Fig. 5A shows an analysis result of a number of the feature selection for establishing a prediction model for grouping hepatocellular carcinoma used to predict whether a metastatic site of the subject patient with hepatocellular carcinoma is in an abdominal cavity.
Fig. 5B shows a receiver operating characteristic curve diagram of the prediction model for grouping hepatocellular carcinoma used to predict whether the metastatic site of the subject patient with hepatocellular carcinoma is in the abdominal cavity before the feature selecting step.
Fig. 5C shows a receiver operating characteristic curve diagram of the prediction model for grouping hepatocellular carcinoma used to predict whether the metastatic site of the subject patient with hepatocellular carcinoma is in the abdominal cavity after the feature selecting step.
Fig. 6A shows an analysis result of a number of the feature selection for establishing a prediction model for grouping hepatocellular carcinoma used to predict whether the subject patient with hepatocellular carcinoma carries hepatitis viruses.
Fig. 6B shows a receiver operating characteristic curve diagram of the prediction model for grouping hepatocellular carcinoma used to predict whether the subject patient with hepatocellular carcinoma carries hepatitis viruses before the feature selecting step.
Fig. 6C shows a receiver operating characteristic curve diagram of the prediction model for grouping hepatocellular carcinoma used to predict whether the subject patient with hepatocellular carcinoma carries hepatitis viruses after the feature selecting step.

### DETAILED DESCRIPTION

Reference will now be made in detail to the present embodiments of the present disclosure, examples of which are illustrated in the accompanying drawings.

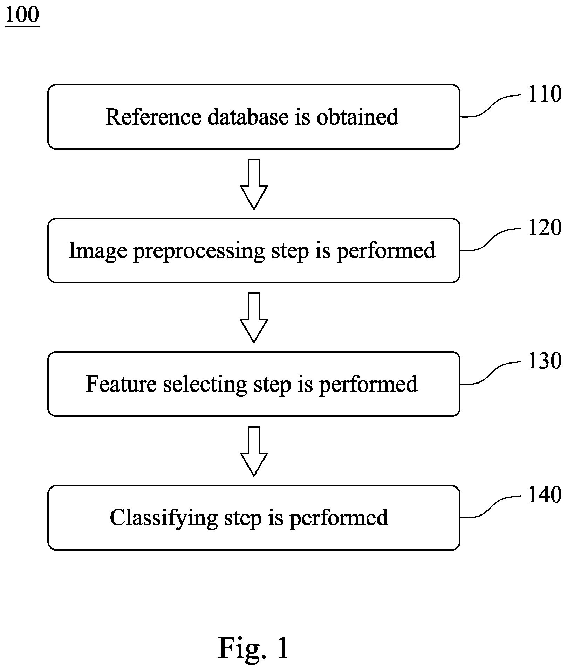

Please refer to Fig. 1, which is a flowchart of establishing steps of a prediction model for grouping hepatocellular carcinoma 100 according to one embodiment of the present disclosure. The establishing steps of a prediction model for grouping hepatocellular carcinoma 100 includes Step 110, Step 120, Step 130 and Step 140. The established prediction model for grouping hepatocellular carcinoma can be used to predict whether a tumor of a subject patient with hepatocellular carcinoma is metastasized, whether a metastatic site of the subject patient with hepatocellular carcinoma is in an abdominal cavity and whether the subject patient with hepatocellular carcinoma carries hepatitis viruses, wherein the hepatitis viruses can be hepatitis B viruses or hepatitis C viruses.

In Step 110, a reference database is obtained, wherein the reference database is established by a plurality of reference arterial phase computed tomographic images of a plurality of reference patients with hepatocellular carcinoma.

In Step 120, an image preprocessing step is performed, wherein the image preprocessing step is for circling a tumor position in each of the reference arterial phase computed tomographic images to obtain reference region of interest images. In liver multi-phase computed tomographic scans without developing agent, liver tumors show iso-hypophyses compared to normal tissues, and both arterial phase and venous phase show hypodense. Therefore, when the reference region of interest images are circled, the tumor boundary can be defined by the change of the image intensity with the blood perfusion and then the tumor position can be manually circled.

In Step 130, a feature selecting step is performed, wherein the feature selecting step is for selecting at least one eigenvalue according to the reference database, the eigenvalue including a texture feature value after analyzing the reference region of interest images by using a Laws filter. The Laws filter is a texture measurement method, which establishes three one-dimensional core vectors representing the intensity (L), the edge (E), and the spot (S), and selects each core vector for convolution in three axial directions to obtain 27 (3³) kinds of calculation results. The calculation result are used to calculate various types of image feature values and the characteristic values of various types of the texture feature values of the images.

When the prediction model for grouping hepatocellular carcinoma is used to predict whether the tumor of the subject patient with hepatocellular carcinoma is metastasized, the texture feature value can include a total gray-level value (TGV), a contrast, a mean, an entropy and a homogeneity of the reference region of interest image. Preferably, the eigenvalue selected in the feature selecting step can further include a homogeneity of the reference region of interest image calculated by a Wavelet filter and a coarseness of the reference region of interest image calculated by a Neighbor Gray Tone Difference Matrix (NGTDM) filter. The wavelet filter uses low-pass filtering and high-pass filtering to disassemble signals in different axial directions to obtain 8 (2³) results. Then the grayscale intensity histogram is obtained from the convolution results. The statistical results are used to calculate the image homogeneity as a characteristic parameter. The Neighbor Gray Tone Difference Matrix filter is used to describe the image intensity varies with space and intensity change. The coarseness is an average difference amount calculated between each voxel and neighboring voxels, which also represents the spatial rate of change. The coarseness indicates a lower spatial rate of change and relatively uniform texture features. Higher coarseness represents lower spatial rate of change and relatively uniform texture feature.

When the prediction model for grouping hepatocellular carcinoma is used to predict whether the metastatic site of the subject patient with hepatocellular carcinoma is in the abdominal cavity, the texture feature value can include the homogeneity and the contrast of the reference region of interest image. Preferably, the eigenvalue selected in the feature selecting step can further include a correlation of the reference region of interest image calculated by a Gary Level Co-Occurrence Matrix (GLCM) filter. The Gary Level Co-Occurrence Matrix filter is a method for analyzing the probability distribution of the gray-scale intensity and the geometric distribution of the gray-scale intensity of the image. The established gray-level co-occurrence matrix is used to calculate the correlation between each voxel and its neighboring voxels, wherein the value of the correlation is between -1 and 1.

When the prediction model for grouping hepatocellular carcinoma is used to predict whether the subject patient with hepatocellular carcinoma carries hepatitis viruses, the texture feature value can include the homogeneity of the reference region of interest image. Preferably, the eigenvalue selected in the feature selecting step can further include a minimum and a homogeneity of the reference region of interest image calculated by a Laplacian of Gaussian (LoG) filter. The Laplacian of Gaussian filter is a band-pass filter that uses the Laplacian operator to process the image and then uses a Gaussian operator to achieve edge enhancement. The values of 0.5 and 1.0 are the standard deviation parameters of the Gaussian curve, and the minimum and the homogeneity are selected according the operation result as the important parameters of the feature.

In Step 140, a classifying step is performed, wherein the classifying step is for achieving a convergence of the eigenvalue by using a supervised learning method to obtain the prediction model for grouping hepatocellular carcinoma. The effectiveness of the supervised learning method is affected by the number of input features and their relevance. The feature selection is mainly to select a subset of the features that help identify results from the original features, and the selected eigenvalue will affect the final classification results. In detail, the at least one eigenvalue and a plurality of reference region of interest images are simultaneously inputted into the support vector machine for achieving the convergence of the eigenvalue to obtain the prediction model for grouping hepatocellular carcinoma. The supervised learning method can be a gap analysis, a support vector machine (SVM), an information gain attribute evaluation (IGAE) or a combination thereof. Preferably, the supervised learning method can be the support vector machine. The established prediction model for grouping hepatocellular carcinoma can predict different conditions of the subject patient with hepatocellular carcinoma through different medical image combinations. In detail, the prediction model for grouping hepatocellular carcinoma can be combined with the data database of the reference patients with hepatocellular carcinoma to predict the conditions of the subject patients more accurately, wherein the data database can include age, gender, medical history and physiological indicators (such as blood pressure, heart rate) of the reference patients.

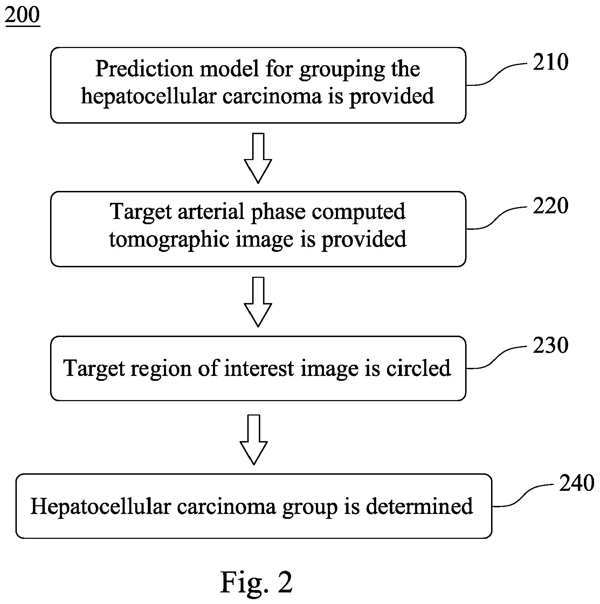

Please refer to Fig. 2, which is a flowchart of a method for determining a hepatocellular carcinoma group 200 according to another embodiment of the present disclosure. The method for determining a hepatocellular carcinoma group 200 includes Step 210, Step 220, Step 230 and Step 240.

In Step 210, the prediction model for grouping hepatocellular carcinoma is provided, wherein the prediction model for grouping hepatocellular carcinoma is established by the aforementioned Steps 110 to 140.

In Step 220, a target arterial phase computed tomographic image of the subject patient with hepatocellular carcinoma is provided.

In Step 230, a tumor position in the target arterial phase computed tomographic image is circled to obtain a target region of interest image. When the target region of interest images are circled, the tumor boundary can be defined by the change of the image intensity with the blood perfusion and then the tumor position can be manually circled.

In Step 240, the prediction model for grouping hepatocellular carcinoma is used to analyze the target region of interest image to determine whether the tumor of the subject patient with hepatocellular carcinoma is metastasized, whether the metastatic position of the subject patient with hepatocellular carcinoma is in the abdominal cavity and whether the subject patient with hepatocellular carcinoma carries hepatitis viruses. An assessment time of the tumor metastasis in the subject patient with hepatocellular carcinoma can be within 5 years. The hepatitis viruses can be hepatitis B viruses or hepatitis C viruses.

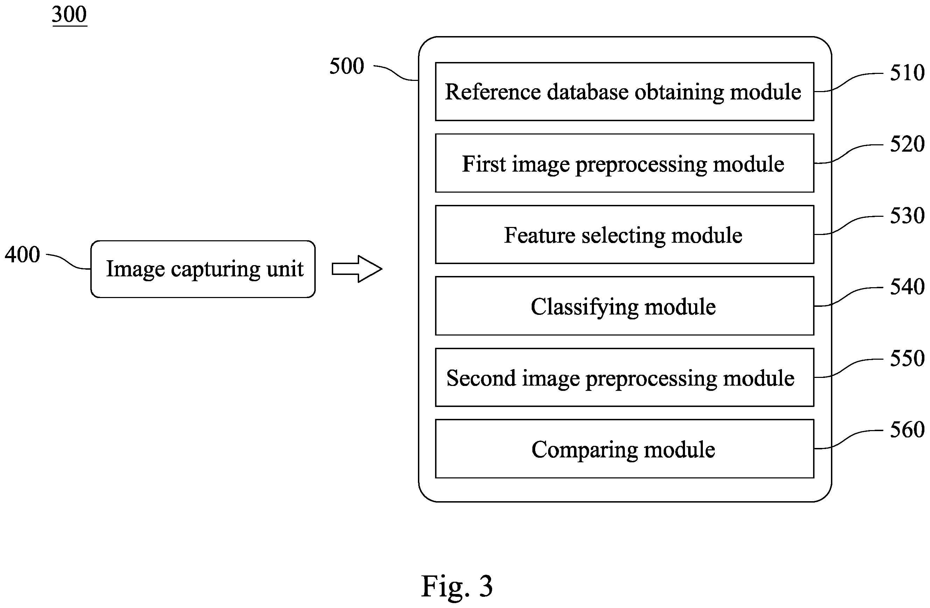

Please refer to Fig. 3, which is a block diagram of a prediction system for grouping hepatocellular carcinoma 300 according to still another embodiment of the present disclosure. The prediction system for grouping hepatocellular carcinoma 300 includes an image capturing unit 400 and a non-transitory machine readable medium 500. The prediction system for grouping hepatocellular carcinoma 300 can be used to predict whether the tumor of the subject patient with hepatocellular carcinoma is metastasized, whether the metastatic site of the subject patient with hepatocellular carcinoma is in the abdominal cavity and whether the subject patient with hepatocellular carcinoma carries hepatitis viruses, wherein the hepatitis viruses can be hepatitis B viruses or hepatitis C viruses.

The image capturing unit 400 is for obtaining the target arterial phase computed tomographic image of the subject patient with hepatocellular carcinoma. The image capturing unit 400 can be a transmissive image scanner, preferably, can be a computed tomography scanner.

The non-transitory machine readable medium 500 storing a program which, when executed by at least one processing unit, predicts hepatocellular carcinoma group of the subject patient with hepatocellular carcinoma. The program includes a reference database obtaining module 510, a first image preprocessing module 520, a feature selecting module 530, a classifying module 540, a second image preprocessing module 550 and a comparing module 560.

The reference database obtaining module 510 is for obtaining a reference database, wherein the reference database is established by a plurality of reference arterial phase computed tomographic images of a plurality of reference patients with hepatocellular carcinoma.

The first image preprocessing module 520 is for circling the tumor position in each of the reference arterial phase computed tomographic images to obtain reference region of interest images.

The feature selecting module 530 is for selecting at least one eigenvalue according to the reference database, wherein the at least one eigenvalue includes a texture feature value after analyzing the reference region of interest images by using the Laws filter. When the prediction system for grouping hepatocellular carcinoma 300 is used to predict whether the tumor of the subject patient with hepatocellular carcinoma is metastasized, the texture feature value can include the total gray-level value, the contrast, the mean, the entropy and the homogeneity of the reference region of interest image. Preferably, the eigenvalue selected by the feature selecting module 530 can further include the homogeneity of the reference region of interest image calculated by the Wavelet filter and the coarseness of the reference region of interest image calculated by the Neighbor Gray Tone Difference Matrix filter. When the prediction system for grouping hepatocellular carcinoma 300 is used to predict whether the metastatic site of the subject patient with hepatocellular carcinoma is in the abdominal cavity, the texture feature value can include the homogeneity and the contrast of the reference region of interest image. Preferably, the eigenvalue selected by the feature selecting module 530 can further include the correlation of the reference region of interest image calculated by the Gary Level Co-Occurrence Matrix filter. When the prediction system for grouping hepatocellular carcinoma 300 is used to predict whether the subject patient with hepatocellular carcinoma carries hepatitis viruses, the texture feature value can include the homogeneity of the reference region of interest image. Preferably, the eigenvalue selected by the feature selecting module 530 can further include the minimum and the homogeneity of the reference region of interest image calculated by the Laplacian of Gaussian filter.

The classifying module 540 is for achieving the convergence of the eigenvalue by using the supervised learning method to obtain the prediction model for grouping hepatocellular carcinoma. The supervised learning method can be the gap analysis, the support vector machine, the information gain attribute evaluation or the combination thereof. Preferably, the supervised learning method can be the support vector machine.

The second image preprocessing module 550 is for circling the tumor position in the target arterial phase computed tomographic image to obtain the target region of interest image. When the target region of interest images are circled, the tumor boundary can be defined by the change of the image intensity with the blood perfusion and then the tumor position can be manually circled.

The comparing module 560 is for analyzing the target region of interest image by the prediction model for grouping hepatocellular carcinoma to determine whether the tumor of the subject patient with hepatocellular carcinoma is metastasized, whether the metastatic site of the subject patient with hepatocellular carcinoma is in the abdominal cavity and whether the subject patient with hepatocellular carcinoma carries hepatitis viruses.

### Examples

### I. Reference database

The reference database used in the present disclosure is the retrospective liver cancer image data collected by the China Medical University Hospital, which includes the arterial phase computed tomographic images of the subjects. This clinical trial program is approved by China Medical University & Hospital Research Ethics Committee, which is numbered as NA/CMUH106-REC1-072. The subjects are patients with hepatocellular carcinoma as the reference patients with hepatocellular carcinoma, and the clinical trial program tracks the conditions of the patients with hepatocellular carcinoma for five years.

### II. Use for predicting whether the tumor of the subject patient with hepatocellular carcinoma is metastasized

In this example, a prediction model for grouping hepatocellular carcinoma used to predict whether the tumor of the subject patient with hepatocellular carcinoma is metastasized needs to be established first. The reference database obtained in Example 1 is established by a plurality of reference arterial phase computed tomographic images of a plurality of reference patients with hepatocellular carcinoma. The reference database is further combined with a data database of the reference patients with hepatocellular carcinoma to select those had the liver tumor metastasis within 5 years. The tumor position in each of the reference arterial phase computed tomographic images is circled according to the image intensity to obtain the reference region of interest images. Then, at least one eigenvalue is selected according to the reference database. The eigenvalue is selected by using the support vector machine, and then a cross-validation is performed through a Stratified K-fold method to determine a number of a feature selection and the selected eigenvalue. The selected eigenvalue and a plurality of reference region of interest images are simultaneously inputted into the support vector machine for achieving the convergence of the eigenvalue to obtain the prediction model for grouping hepatocellular carcinoma used to predict whether the tumor of the subject patient with hepatocellular carcinoma is metastasized.

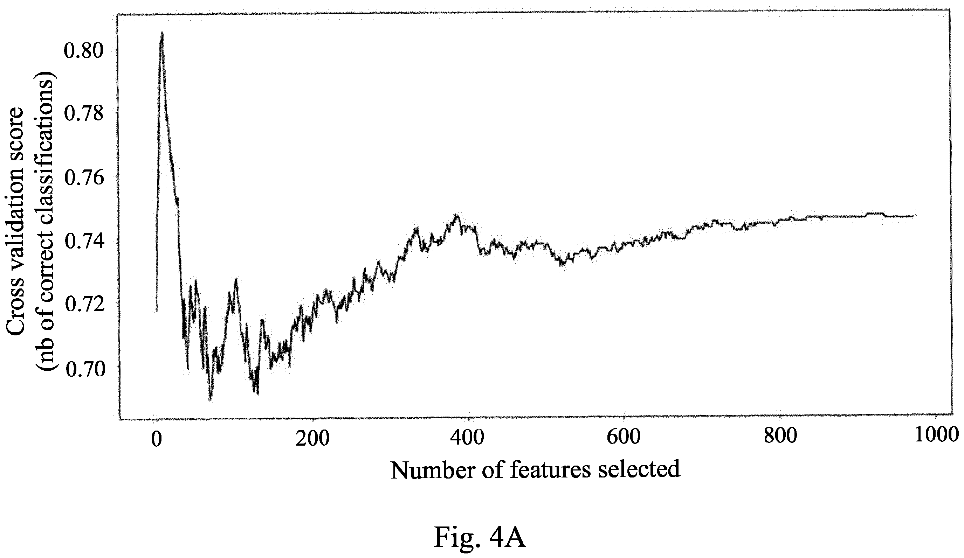

Please refer to Fig. 4A, which shows an analysis result of the number of the feature selection for establishing the prediction model for grouping hepatocellular carcinoma used to predict whether the tumor of the subject patient with hepatocellular carcinoma is metastasized. In Fig. 4A, when the number of the feature selection is 10, the best cross-validation score (number of correct classifications) is obtained. Therefore, the number of the selected eigenvalues is 10 for establishing the prediction model for grouping hepatocellular carcinoma used to predict whether the tumor of the subject patient with hepatocellular carcinoma is metastasized. In detail, the selected eigenvalues are the total gray-level value, the contrast, the mean, the entropy and the homogeneity of the reference region of interest image analyzed by the Laws filter, the homogeneity of the reference region of interest image calculated by the Wavelet filter, and the coarseness of the reference region of interest image calculated by the Neighbor Gray Tone Difference Matrix filter. Preferably, the selected eigenvalues are Laws_EEE_TGV, Laws_ESE_TGV, Laws_LLS_TGV, Laws_LSS_TGV, Laws_ESE_contrast, Laws_EES_mean, Laws_LSL_entropy, Laws_SLL_homogeneity, wavelet_LLH_homogeneity and NGTDM_Coarseness. Law represents the Laws filter, wavelet represents the Wavelet filter, NGTDM represents the Neighbor Gray Tone Difference Matrix filter, and TGV represents the total gray-level value. In selected eigenvalues analyzed by the Laws filter, E/S/L represents the parameters used, respectively, wherein L represents the intensity, E represents the edge, and S represents the spot. In selected eigenvalue calculated by the Wavelet filter, L/H represents the parameters used, respectively, wherein L represents low-pass filtering and H represents high-pass filtering.

The established prediction model for grouping hepatocellular carcinoma is further used to predict whether the tumor of the subject patient with hepatocellular carcinoma is metastasized including following steps. The prediction model for grouping hepatocellular carcinoma used to predict whether the tumor of the subject patient with hepatocellular carcinoma is metastasized is provided. The target arterial phase computed tomographic image of the subject patient with hepatocellular carcinoma is provided. The tumor position in the target arterial phase computed tomographic image is circled to obtain the target region of interest image. The prediction model for grouping hepatocellular carcinoma used to predict whether the tumor of the subject patient with hepatocellular carcinoma is metastasized is used to analyze the target region of interest image to determine whether the tumor of the subject patient with hepatocellular carcinoma is metastasized. The criterion for the determination is to compare the similarity between the data of the prediction model for grouping hepatocellular carcinoma used to predict whether the tumor of the subject patient with hepatocellular carcinoma is metastasized and the reference database, and a probability value for each subject patient with hepatocellular carcinoma is given at different endpoint.

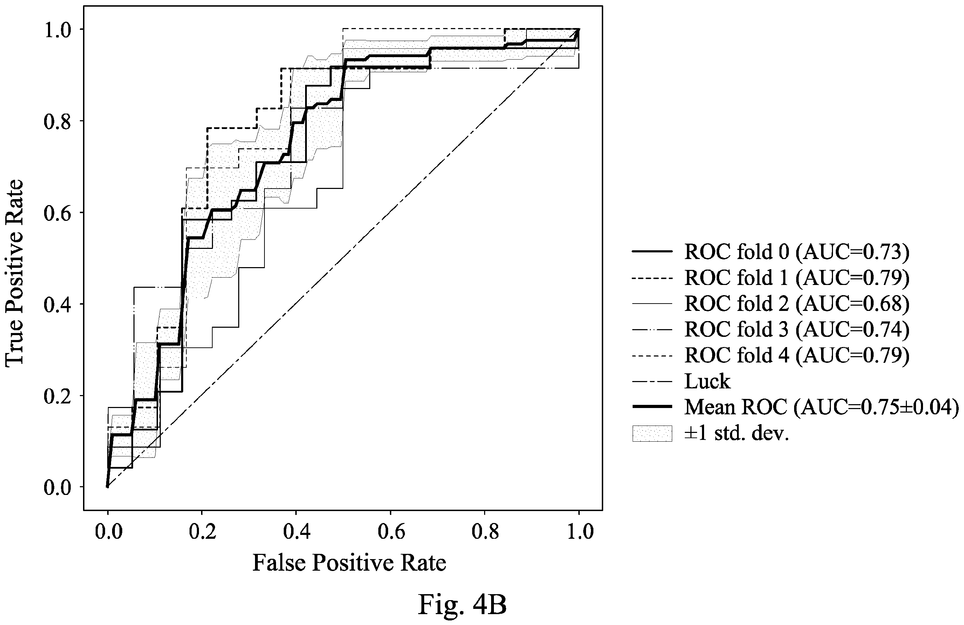

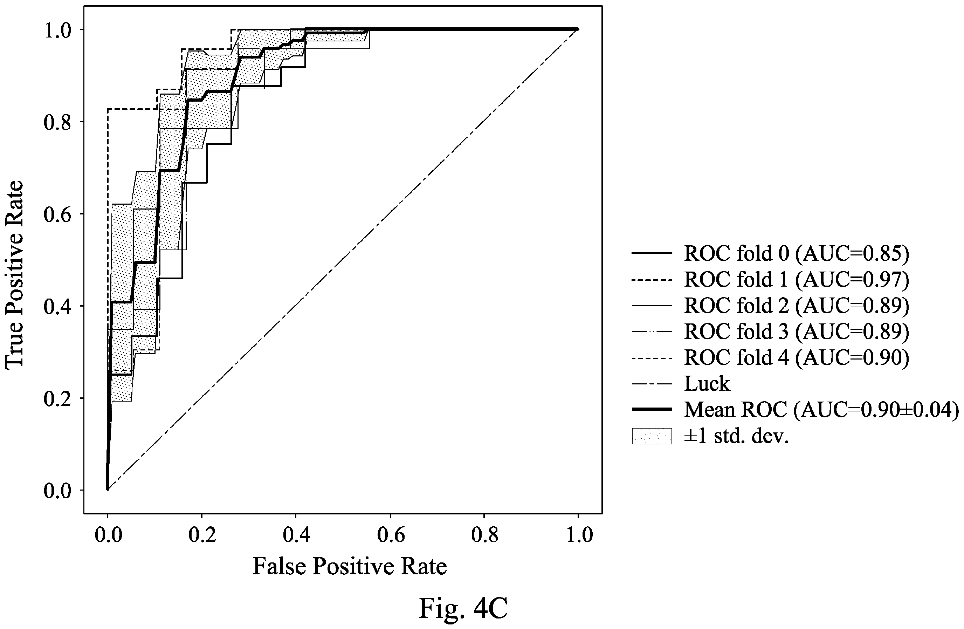

Please refer to Figs. 4B and 4C. Fig. 4B shows a receiver operating characteristic curve (ROC) diagram of the prediction model for grouping hepatocellular carcinoma used to predict whether the tumor of the subject patient with hepatocellular carcinoma is metastasized before the feature selecting step, wherein a transfer classification is performed by using 983 eigenvalues. Fig. 4C shows a receiver operating characteristic curve diagram of the prediction model for grouping hepatocellular carcinoma used to predict whether the tumor of the subject patient with hepatocellular carcinoma is metastasized after the feature selecting step, wherein the transfer classification is performed by using the 10 selected eigenvalues. In Fig. 4B, when the prediction model for grouping hepatocellular carcinoma used to predict whether the tumor of the subject patient with hepatocellular carcinoma is metastasized established before the feature selecting step is used to predict whether the tumor of the subject patient with hepatocellular carcinoma is metastasized within 5 years, the average Area under the Curve (AUC) is 75 %. In Fig. 4C, when the prediction model for grouping hepatocellular carcinoma used to predict whether the tumor of the subject patient with hepatocellular carcinoma is metastasized of the present disclosure is used to predict whether the tumor of the subject patient with hepatocellular carcinoma is metastasized within 5 years, the average AUC can be increased to 90 %. The result indicates that the prediction model for grouping hepatocellular carcinoma of the present disclosure can be used to accurately predict whether the tumor of the subject patient with hepatocellular carcinoma is metastasized within 5 years.

### III. Use for predicting whether the metastatic site of the subject patient with hepatocellular carcinoma is in the abdominal cavity

In this example, a prediction model for grouping hepatocellular carcinoma used to predict whether the metastatic site of the subject patient with hepatocellular carcinoma is in the abdominal cavity needs to be established first. The reference database obtained in Example 1 is established by a plurality of reference arterial phase computed tomographic images of a plurality of reference patients with hepatocellular carcinoma. The reference database is further combined with the data database of the reference patients with hepatocellular carcinoma to select those had the liver tumor metastasis in the abdomen. The tumor position in each of the reference arterial phase computed tomographic images is circled according to the image intensity to obtain the reference region of interest images. Then, at least one eigenvalue is selected according to the reference database. The eigenvalue is selected by using the support vector machine, and then the cross-validation is performed through the Stratified K-fold method to determine the number of the feature selection and the selected eigenvalue. The selected eigenvalue and a plurality of reference region of interest images are inputted into the support vector machine for achieving the convergence of the eigenvalue to obtain the prediction model for grouping hepatocellular carcinoma used to predict whether the metastatic site of the subject patient with hepatocellular carcinoma is in the abdominal cavity.

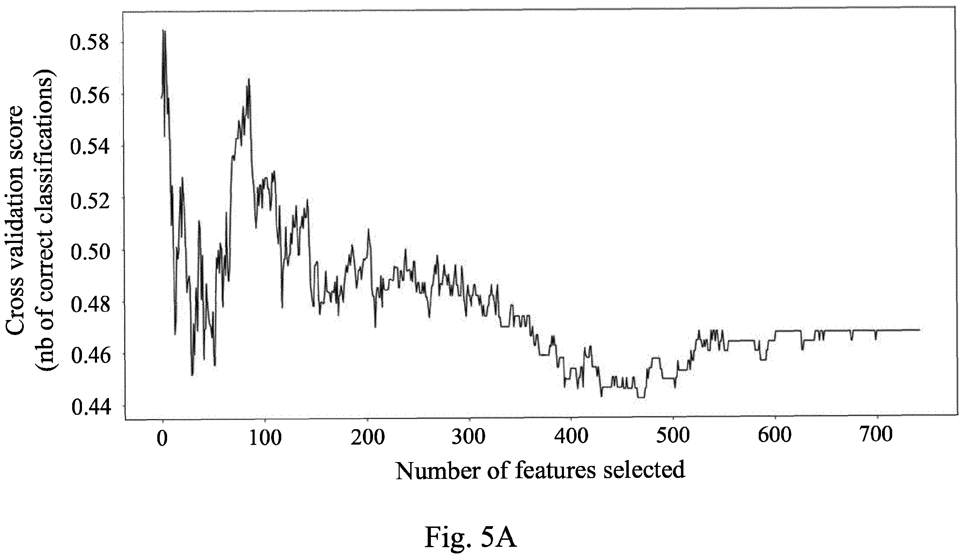

Please refer to Fig. 5A, which shows an analysis result of the number of the feature selection for establishing the prediction model for grouping hepatocellular carcinoma used to predict whether the metastatic site of the subject patient with hepatocellular carcinoma is in the abdominal cavity. In Fig. 5A, when the number of the feature selection is 3, the best cross-validation score (number of correct classifications) is obtained. Therefore, the number of the selected eigenvalues is 3 for establishing the prediction model for grouping hepatocellular carcinoma used to predict whether the metastatic site of the subject patient with hepatocellular carcinoma is in the abdominal cavity. In detail, the selected eigenvalues are the homogeneity and the contrast of the reference region of interest image analyzed by the Laws filter, and the correlation of the reference region of interest image calculated by the Gary Level Co-Occurrence Matrix filter. Preferably, the selected eigenvalues are Laws_ELS_homogeneity, Laws_SEE_contrast and GLCM_Correlation. Law represents the Laws filter and GLCM represents the Gary Level Co-Occurrence Matrix filter. In selected eigenvalues analyzed by the Laws filter, E/S/L represents the parameters used, respectively, wherein L represents the intensity, E represents the edge, and S represents the spot.

The established prediction model for grouping hepatocellular carcinoma is further used to predict whether the metastatic site of the subject patient with hepatocellular carcinoma is in the abdominal cavity including following steps. The prediction model for grouping hepatocellular carcinoma used to predict whether the metastatic site of the subject patient with hepatocellular carcinoma is in the abdominal cavity is provided. The target arterial phase computed tomographic image of the subject patient with hepatocellular carcinoma is provided. The tumor position in the target arterial phase computed tomographic image is circled to obtain the target region of interest image. The prediction model for grouping hepatocellular carcinoma used to predict whether the metastatic site of the subject patient with hepatocellular carcinoma is in the abdominal cavity is used to analyze the target region of interest image to determine whether the metastatic site of the subject patient with hepatocellular carcinoma is in the abdominal cavity. The criterion for the determination is to compare the similarity between the data of the prediction model for grouping hepatocellular carcinoma used to predict whether the metastatic site of the subject patient with hepatocellular carcinoma is in the abdominal cavity and the reference database, and the probability value for each subject patient with hepatocellular carcinoma is given at different endpoint.

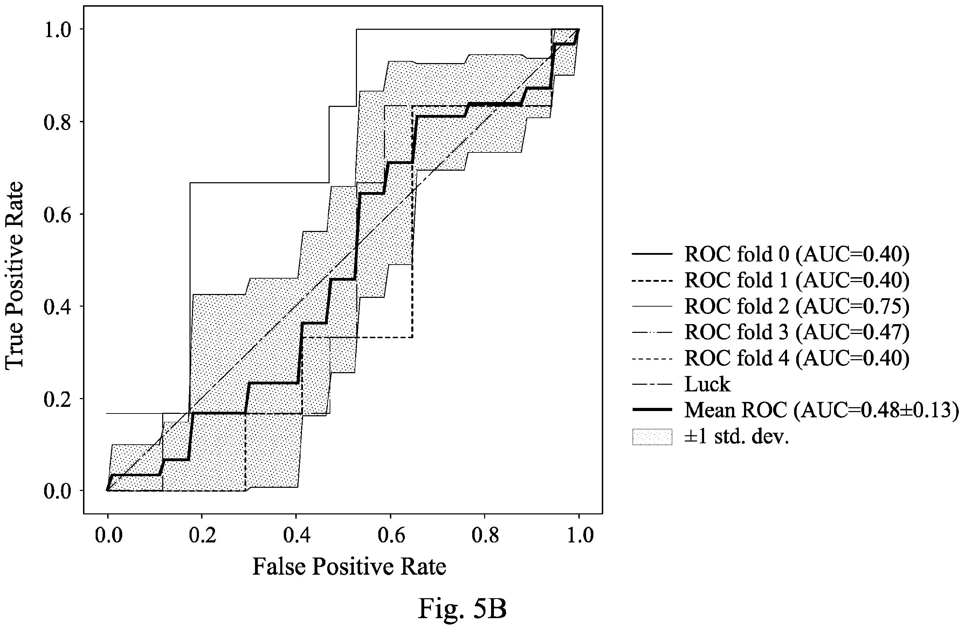

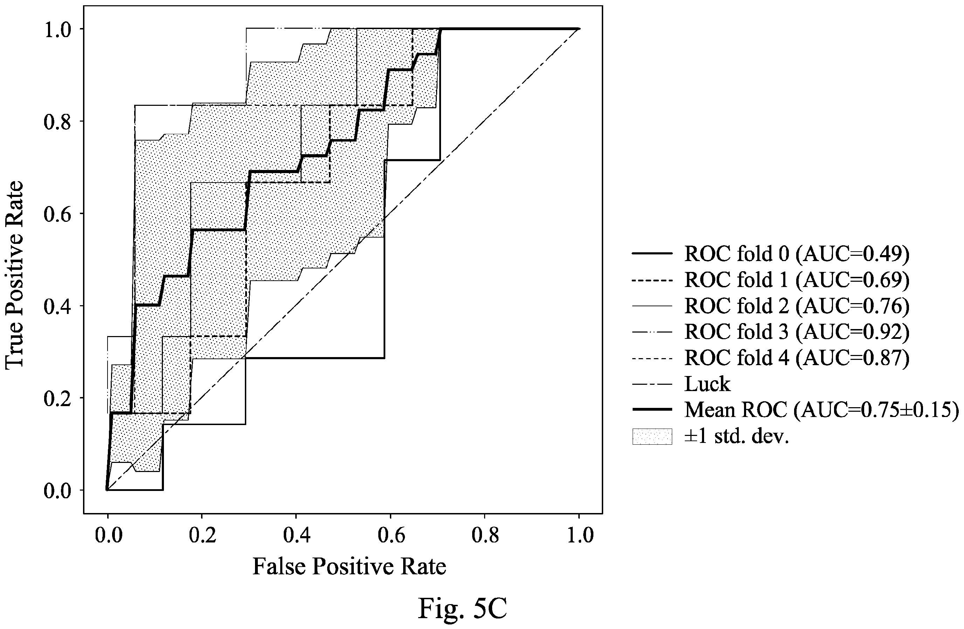

Please refer to Figs. 5B and 5C. Fig. 5B shows a receiver operating characteristic curve diagram of the prediction model for grouping hepatocellular carcinoma used to predict whether the metastatic site of the subject patient with hepatocellular carcinoma is in the abdominal cavity before the feature selecting step, wherein the transfer classification is performed by using 743 eigenvalues. Fig. 5C shows a receiver operating characteristic curve diagram of the prediction model for grouping hepatocellular carcinoma used to predict whether the metastatic site of the subject patient with hepatocellular carcinoma is in the abdominal cavity after the feature selecting step, wherein the transfer classification is performed by using the 3 selected eigenvalues. In Fig. 5B, when the prediction model for grouping hepatocellular carcinoma used to predict whether the metastatic site of the subject patient with hepatocellular carcinoma is in the abdominal cavity established before the feature selecting step is used to predict whether the metastatic site of the subject patient with hepatocellular carcinoma is in the abdominal cavity, the average AUC is 48 %. In Fig. 5C, when the prediction model for grouping hepatocellular carcinoma used to predict whether the metastatic site of the subject patient with hepatocellular carcinoma is in the abdominal cavity of the present disclosure is used to predict whether the metastatic site of the subject patient with hepatocellular carcinoma is in the abdominal cavity, the average AUC can be increased to 75 %. The result indicates that the prediction model for grouping hepatocellular carcinoma of the present disclosure can be used to accurately predict whether the metastatic site of the subject patient with hepatocellular carcinoma is in the abdominal cavity.

### IV. Use for predicting whether the subject patient with hepatocellular carcinoma carries hepatitis viruses

In this example, the prediction model for grouping hepatocellular carcinoma used to predict whether the subject patient with hepatocellular carcinoma carries hepatitis viruses needs to be established first. The reference database obtained in Example 1 is established by a plurality of reference arterial phase computed tomographic images of a plurality of reference patients with hepatocellular carcinoma. The reference database is further combined with the data database of the reference patients with hepatocellular carcinoma to select those carried hepatitis B viruses or hepatitis C viruses. The tumor position in each of the reference arterial phase computed tomographic images is circled according to the image intensity to obtain the reference region of interest images. Then, at least one eigenvalue is selected according to the reference database. The eigenvalue is selected by using the support vector machine, and then the cross-validation is performed through the Stratified K-fold method to determine the number of the feature selection and the selected eigenvalue. The selected eigenvalue and a plurality of reference region of interest images are inputted into the support vector machine for achieving the convergence of the eigenvalue to obtain the prediction model for grouping hepatocellular carcinoma used to predict whether the subject patient with hepatocellular carcinoma carries hepatitis viruses.

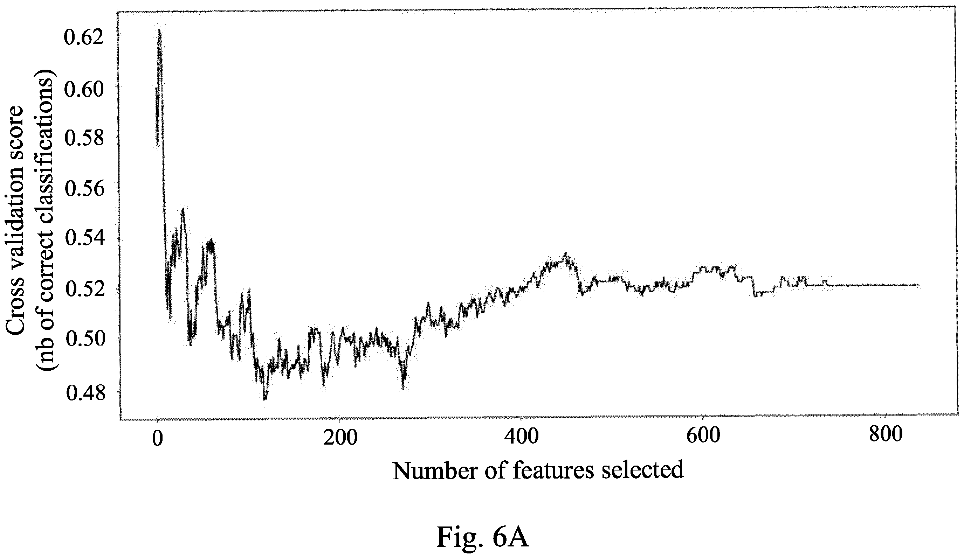

Please refer to Fig. 6A, which shows an analysis result of the number of the feature selection for establishing the prediction model for grouping hepatocellular carcinoma used to predict whether the subject patient with hepatocellular carcinoma carries hepatitis viruses. In Fig. 6A, when the number of the feature selection is 5, the best cross-validation score (number of correct classifications) is obtained. Therefore, the number of the selected eigenvalues is 5 for establishing the prediction model for grouping hepatocellular carcinoma used to predict whether the subject patient with hepatocellular carcinoma carries hepatitis viruses. In detail, the selected eigenvalues are the homogeneity of the reference region of interest image analyzed by the Laws filter, and the minimum and the homogeneity of the reference region of interest image calculated by the Laplacian of Gaussian filter. Preferably, the selected eigenvalues are Laws_ELE_homogeneity, Laws_LSE_homogeneity, Laws_SEL_homogeneity, LoG_1_homogeneity and LoG_0.5_min. Law represents the Laws filter, LoG represents the Laplacian of Gaussian filter, and min represents the minimum. In selected eigenvalues analyzed by the Laws filter, E/S/L represents the parameters used, respectively, wherein L represents the intensity, E represents the edge, and S represents the spot. In selected eigenvalue calculated by the Laplacian of Gaussian filter, 0.5/1.0 represents the standard deviation parameter of the Gaussian curve, respectively.

The established prediction model for grouping hepatocellular carcinoma is further used to predict whether the subject patient with hepatocellular carcinoma carries hepatitis viruses including following steps. The prediction model for grouping hepatocellular carcinoma used to predict whether the subject patient with hepatocellular carcinoma carries hepatitis viruses is provided. The target arterial phase computed tomographic image of the subject patient with hepatocellular carcinoma is provided. The tumor position in the target arterial phase computed tomographic image is circled to obtain the target region of interest image. The prediction model for grouping hepatocellular carcinoma used to predict whether the subject patient with hepatocellular carcinoma carries hepatitis viruses is used to analyze the target region of interest image to determine whether the subject patient with hepatocellular carcinoma carries hepatitis viruses. The criterion for the determination is to compare the similarity between the data of the prediction model for grouping hepatocellular carcinoma used to predict whether the subject patient with hepatocellular carcinoma carries hepatitis viruses and the reference database, and a probability value for each subject patient with hepatocellular carcinoma is given at different endpoint.

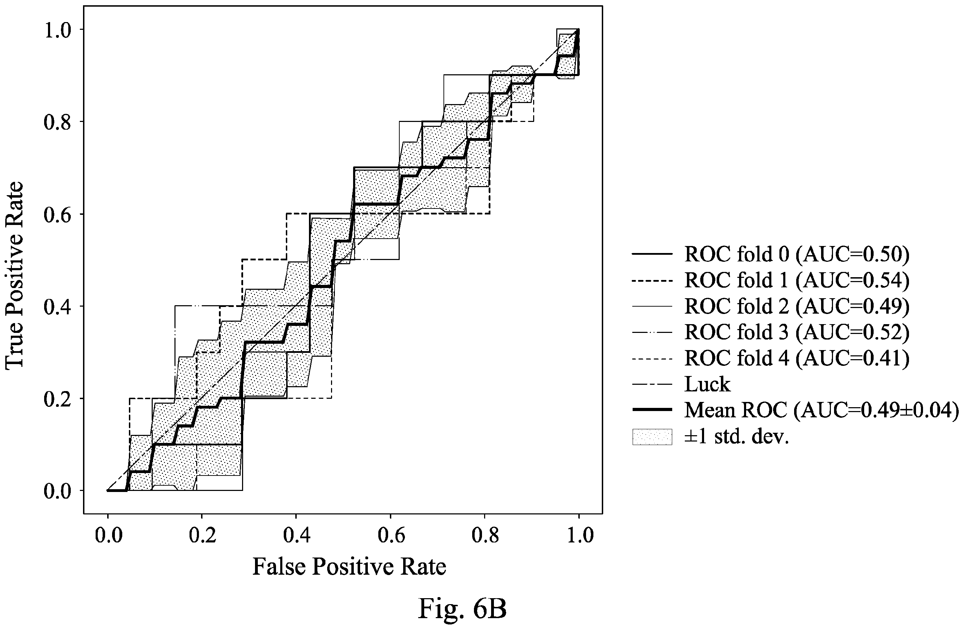

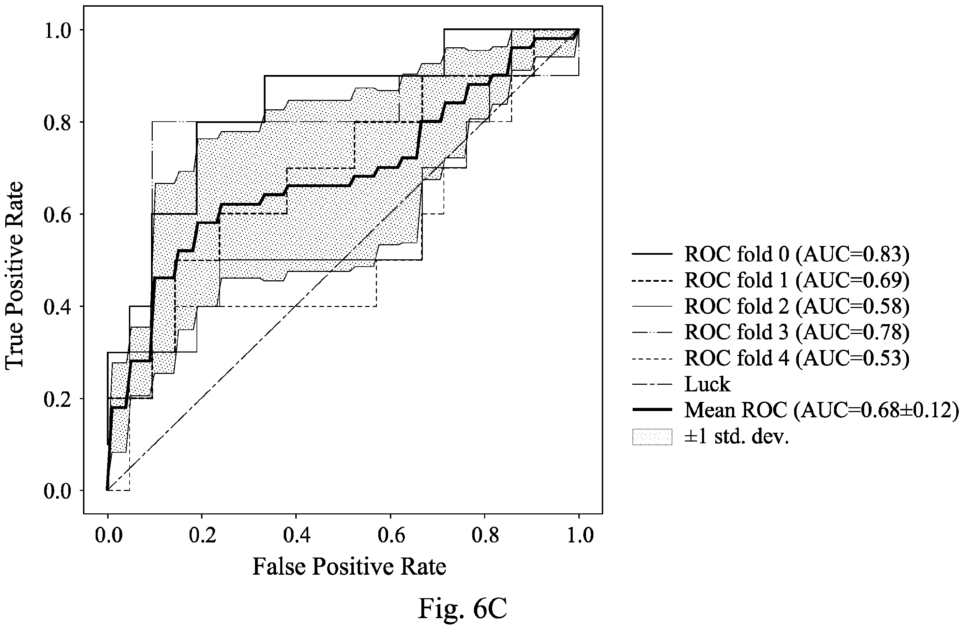

Please refer to Figs. 6B and 6C. Fig. 6B shows a receiver operating characteristic curve diagram of the prediction model for grouping hepatocellular carcinoma used to predict whether the subject patient with hepatocellular carcinoma carries hepatitis viruses before the feature selecting step, wherein the transfer classification is performed by using 839 eigenvalues. Fig. 6C shows a receiver operating characteristic curve diagram of the prediction model for grouping hepatocellular carcinoma used to predict whether the subject patient with hepatocellular carcinoma carries hepatitis viruses after the feature selecting step, the transfer classification is performed by using the 5 selected eigenvalues. In Fig. 6B, when the prediction model for grouping hepatocellular carcinoma used to predict whether the subject patient with hepatocellular carcinoma carries hepatitis viruses established before the feature selecting step is used to predict whether the subject patient with hepatocellular carcinoma carries hepatitis viruses, the average AUC is 49 %. In Fig. 6C, when the prediction model for grouping hepatocellular carcinoma used to predict whether the subject patient with hepatocellular carcinoma carries hepatitis viruses of the present disclosure is used to predict whether the subject patient with hepatocellular carcinoma carries hepatitis viruses, the average AUC can be increased to 68 %. The result indicates that the prediction model for grouping hepatocellular carcinoma of the present disclosure can be used to accurately predict whether the subject patient with hepatocellular carcinoma carries hepatitis viruses.

To sum up, the prediction model for grouping hepatocellular carcinoma, the prediction system for grouping hepatocellular carcinoma and the method for determining hepatocellular carcinoma group are provided in the present disclosure, which can use medical imaging data from individual cases to perform automated and rapid data analysis for assisting the medical personnel to perform the interpretation and confirm the diagnosis as soon as possible in the early stage of hepatocellular carcinoma with insignificant symptoms. In addition, the prediction model for grouping hepatocellular carcinoma, the prediction system for grouping hepatocellular carcinoma and the method for determining hepatocellular carcinoma group of the present disclosure can assist in quantifying tumor information and assist the clinician in determining the treatment strategy. The treatment strategies of hepatocellular carcinoma include local treatment and systemic treatment based on patient's conditions. Further treatments may include surgical resection, transcatheter embolization, chemotherapy, radiation therapy, target drugs, and immunotherapy, wherein therapeutic costs and concurrent side effects of immunotherapy and target drug are higher than those of other treatments. The prediction model for grouping hepatocellular carcinoma, the prediction system for grouping hepatocellular carcinoma and the method for determining hepatocellular carcinoma group of the present disclosure can assist in understanding tumor characteristics, which can not only assist the doctor in formulating the therapeutic strategy, but also can help the patient to reduce the treatment cost and concurrent side-effect risk.

Furthermore, the prediction model for grouping hepatocellular carcinoma, the prediction system for grouping hepatocellular carcinoma and the method for determining hepatocellular carcinoma group of the present disclosure can predict whether the tumor will metastasize and its metastatic site through high probability. If the risk of metastasis is high, the subject can further perform detailed examinations based on the predicted metastatic site. The subject also can regularly track or perform preventative treatment and appropriate care prevention for high risk metastatic site to effectively reduce healthcare costs and care expenditure.

The prediction model for grouping hepatocellular carcinoma, the prediction system for grouping hepatocellular carcinoma and the method for determining hepatocellular carcinoma group of the present disclosure can also improve inspection accuracy, reduce detection items and reduce patient discomfort by non-intrusive inspection. For example, if the subject patient with hepatocellular carcinoma carries hepatitis B virus or hepatitis C virus, the aforementioned treatment strategies need to cooperate with antiviral drugs for treatment assistance. Therefore, the prediction model for grouping hepatocellular carcinoma, the prediction system for grouping hepatocellular carcinoma and the method for determining hepatocellular carcinoma group of the present disclosure can be used as reference for medications of doctors and assessment of therapeutic effect the patient, assist doctors in accurate medication, shorten trial period of the patient and reduce discomfort of the patient.

## Claims

1. A prediction model for grouping a hepatocellular carcinoma, comprising following establishing steps (100):
obtaining a reference database (110), wherein the reference database is established by a plurality of reference arterial phase computed tomographic images of a plurality of reference patients with a hepatocellular carcinoma;
performing an image preprocessing step (120), wherein the image preprocessing step is for circling a tumor position in each of the reference arterial phase computed tomographic images to obtain reference region of interest images;
performing a feature selecting step (130), wherein the feature selecting step is for selecting at least one eigenvalue according to the reference database, and the eigenvalue comprising a texture feature value after analyzing the reference region of interest images by using a Laws filter; and
performing a classifying step (140), wherein the classifying step is for achieving a convergence of the eigenvalue by using a supervised learning method to obtain the prediction model for grouping the hepatocellular carcinoma;
wherein the prediction model for grouping the hepatocellular carcinoma is used to predict whether a tumor of a subject patient with the hepatocellular carcinoma is metastasized, whether a metastatic site of the subject patient with the hepatocellular carcinoma is in an abdominal cavity and whether the subject patient with the hepatocellular carcinoma carries hepatitis viruses.

2. The prediction model for grouping the hepatocellular carcinoma of claim 1, wherein the supervised learning method is a support vector machine.

3. The prediction model for grouping the hepatocellular carcinoma of claim 1, wherein when the prediction model for grouping the hepatocellular carcinoma is used to predict whether the tumor of the subject patient with the hepatocellular carcinoma is metastasized, the texture feature value comprises a total gray-level value (TGV), a contrast, a mean, an entropy and a homogeneity of the reference region of interest image.

4. The prediction model for grouping the hepatocellular carcinoma of claim 3, wherein when the prediction model for grouping the hepatocellular carcinoma is used to predict whether the tumor of the subject patient with the hepatocellular carcinoma is metastasized, the eigenvalue selected in the feature selecting step (130) further comprises:
a homogeneity of the reference region of interest image calculated by a Wavelet filter; and
a coarseness of the reference region of interest image calculated by a Neighbor Gray Tone Difference Matrix (NGTDM) filter.

5. The prediction model for grouping the hepatocellular carcinoma of claim 1, wherein when the prediction model for grouping the hepatocellular carcinoma is used to predict whether the metastatic site of the subject patient with the hepatocellular carcinoma is in the abdominal cavity, the texture feature value comprises a homogeneity and a contrast of the reference region of interest image.

6. The prediction model for grouping the hepatocellular carcinoma of claim 5, wherein when the prediction model for grouping the hepatocellular carcinoma is used to predict whether the metastatic site of the subject patient with the hepatocellular carcinoma is in the abdominal cavity, the eigenvalue selected in the feature selecting step (130) further comprises a correlation of the reference region of interest image calculated by a Gary Level Co-Occurrence Matrix (GLCM) filter.

7. The prediction model for grouping the hepatocellular carcinoma of claim 1, wherein the hepatitis viruses are hepatitis B viruses or hepatitis C viruses.

8. The prediction model for grouping the hepatocellular carcinoma of claim 7, wherein when the prediction model for grouping the hepatocellular carcinoma is used to predict whether the subject patient with the hepatocellular carcinoma carries hepatitis viruses, the texture feature value comprises a homogeneity of the reference region of interest image.

9. The prediction model for grouping the hepatocellular carcinoma of claim 8, wherein when the prediction model for grouping the hepatocellular carcinoma is used to predict whether the subject patient with the hepatocellular carcinoma carries hepatitis viruses, the eigenvalue selected in the feature selecting step (130) further comprises a minimum and a homogeneity of the reference region of interest image calculated by a Laplacian of Gaussian (LoG) filter.

10. A method for determining a hepatocellular carcinoma group (200), comprising:
providing the prediction model for grouping the hepatocellular carcinoma of claim 1 (210);
providing a target arterial phase computed tomographic image of a subject patient with the hepatocellular carcinoma (220);
circling a tumor position in the target arterial phase computed tomographic image to obtain a target region of interest image (230); and
using the prediction model for grouping the hepatocellular carcinoma to analyze the target region of interest image to determine whether a tumor of the subject patient with the hepatocellular carcinoma is metastasized, whether the metastatic position of the subject patient with the hepatocellular carcinoma is in an abdominal cavity and whether the subject patient with the hepatocellular carcinoma carries hepatitis viruses (240).

11. The method for determining hepatocellular carcinoma group (200) of claim 10, wherein an assessment time of a tumor metastasis in the subject patient with the hepatocellular carcinoma is within 5 years.

12. The method for determining hepatocellular carcinoma group (200) of claim 10, wherein the hepatitis viruses are hepatitis B viruses or hepatitis C viruses.

13. A prediction system for grouping a hepatocellular carcinoma (300), comprising:
an image capturing unit (400) for obtaining a target arterial phase computed tomographic image of a subject patient with a hepatocellular carcinoma; and
a non-transitory machine readable medium (500) storing a program, which when executed by at least one processing unit, predicts a hepatocellular carcinoma group of the subject patient with the hepatocellular carcinoma, the program comprising:
a reference database obtaining module (510) for obtaining a reference database, wherein the reference database is established by a plurality of reference arterial phase computed tomographic images of a plurality of reference patients with the hepatocellular carcinoma;
a first image preprocessing module (520) for circling a tumor position in each of the reference arterial phase computed tomographic images to obtain reference region of interest images;
a feature selecting module (530) for selecting at least one eigenvalue according to the reference database, wherein the eigenvalue comprises a texture feature value after analyzing the reference region of interest images by using a Laws filter;
a classifying module (540) for achieving a convergence of the eigenvalue by using a supervised learning method to obtain a prediction model for grouping the hepatocellular carcinoma;
a second image preprocessing module (550) for circling a tumor position in the target arterial phase computed tomographic image to obtain a target region of interest image; and
a comparing module (560) for analyzing the target region of interest image by the prediction model for grouping the hepatocellular carcinoma to determine whether a tumor of the subject patient with the hepatocellular carcinoma is metastasized, whether a metastatic site of the subject patient with the hepatocellular carcinoma is in an abdominal cavity and whether the subject patient with the hepatocellular carcinoma carries hepatitis viruses.

14. The prediction system for grouping hepatocellular carcinoma (300) of claim 13, wherein when the prediction system for grouping the hepatocellular carcinoma (300) is used to determine whether the tumor of the subject patient with the hepatocellular carcinoma is metastasized, the texture feature value comprises a total gray-level value (TGV), a contrast, a mean, an entropy and a homogeneity of the reference region of interest image.

15. The prediction system for grouping hepatocellular carcinoma (300) of claim 14, wherein when the prediction system for grouping the hepatocellular carcinoma (300) is used to determine whether the tumor of the subject patient with the hepatocellular carcinoma is metastasized, the eigenvalue selected by the feature selecting module (530) further comprises:
a homogeneity of the reference region of interest image calculated by a Wavelet filter; and
a coarseness of the reference region of interest image calculated by a Neighbor Gray Tone Difference Matrix (NGTDM) filter.

16. The prediction system for grouping hepatocellular carcinoma (300) of claim 13, wherein when the prediction system for grouping the hepatocellular carcinoma (300) is used to determine whether the metastatic site of the subject patient with the hepatocellular carcinoma is in the abdominal cavity, the texture feature value comprises a homogeneity and a contrast of the reference region of interest image.

17. The prediction system for grouping hepatocellular carcinoma (300) of claim 16, wherein when the prediction system for grouping the hepatocellular carcinoma (300) is used to determine whether the metastatic site of the subject patient with the hepatocellular carcinoma is in the abdominal cavity, the eigenvalue selected by the feature selecting module (530) further comprises a correlation of the reference region of interest image calculated by a Gary Level Co-Occurrence Matrix (GLCM) filter.

18. The prediction system for grouping hepatocellular carcinoma (300) of claim 13, wherein the hepatitis viruses are hepatitis B viruses or hepatitis C viruses.

19. The prediction system for grouping hepatocellular carcinoma (300) of claim 18, wherein when the prediction system for grouping the hepatocellular carcinoma (300) is used to determine whether the subject patient with the hepatocellular carcinoma carries hepatitis viruses, the texture feature value comprises a homogeneity of the reference region of interest image.

20. The prediction system for grouping hepatocellular carcinoma (300) of claim 18, wherein when the prediction system for grouping the hepatocellular carcinoma (300) is used to determine whether the subject patient with the hepatocellular carcinoma carries hepatitis viruses, the eigenvalue selected by the feature selecting module (530) further comprises a minimum and a homogeneity of the reference region of interest image calculated by a Laplacian of Gaussian (LoG) filter.
